(19) Europäisches Patentamt / European Patent Office / Office européen des brevets

(11) **EP 3 810 267 B1**

(12) **EUROPEAN PATENT SPECIFICATION**

(45) Date of publication and mention of the grant of the patent:
**07.12.2022 Bulletin 2022/49**

(21) Application number: **19744855.8**

(22) Date of filing: **19.06.2019**

(51) International Patent Classification (IPC):
*A61N 5/10* (2006.01)    *H05H 13/00* (2006.01)
*H05H 13/04* (2006.01)    *H05H 9/00* (2006.01)
*H05H 7/04* (2006.01)    *H05H 13/02* (2006.01)

(52) Cooperative Patent Classification (CPC):
**A61N 5/1043; H05H 7/001; H05H 7/04;**
A61N 2005/1087; H05H 9/041; H05H 2007/002;
H05H 2007/046; H05H 2277/11

(86) International application number:
**PCT/IB2019/055167**

(87) International publication number:
**WO 2019/244073 (26.12.2019 Gazette 2019/52)**

(54) **BEAM TRANSPORT LINE FOR RADIOTHERAPY SYSTEMS AND RADIOTHERAPY SYSTEM WITH BEAM TRANSPORT LINE**

STRAHLTRANSPORTLEITUNG FÜR STRAHLENTHERAPIESYSTEME UND STRAHLENTHERAPIESYSTEM MIT STRAHLTRANSPORTLEITUNG

LIGNE DE TRANSPORT DE FAISCEAU POUR SYSTÈMES DE RADIOTHÉRAPIE ET SYSTÈME DE RADIOTHÉRAPIE ASSOCIÉ

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **19.06.2018 IT 201800006452**

(43) Date of publication of application:
**28.04.2021 Bulletin 2021/17**

(73) Proprietor: **Advanced Oncotherapy PLC**
**London EC2M 1QS (GB)**

(72) Inventor: **AMALDI, Ugo**
**1223 Geneva (CH)**

(74) Representative: **Di Bernardo, Antonio**
**Thinx S.r.l.**
**P.le Luigi Cadorna, 10**
**20123 Milano (IT)**

(56) References cited:
**EP-A1- 3 178 522**    **US-A1- 2010 012 859**

## Description

### TECHNICAL FIELD

The present invention relates to a particle beam transport line for a radiotherapy system and to a radiotherapy system with beam transport line.

The invention particularly relates to a beam transport line with rapidly varying 2-quadrant or 4-quadrant power supplies to perform a "Fast Adaptive Spot Scanning Therapy" (FASST) with protons or light ions beams, according to the preamble to Claim 1.

### BACKGROUND ART

Radiotherapy is a known method for treating, primarily, cancer, but also other focal pathologies for example arrhythmias, arteriovenous and other malformations, renal denervation, keloid scars etc.

A typical radiotherapy system includes a source of radiation and some means to direct the radiation to the patient. Forms of radiation typically used include gamma rays emitted by radioactive sources, high energy X-rays, electrons, protons and other light ions, in particular carbon ions.

Proton radiotherapy and therapy with other light ions (collectively called "hadrons") require some source of protons plus instruments to accelerate the protons and form a medically useful beam. The accelerating instruments can typically be cyclotrons, synchrocyclotrons, synchrotrons, or linear accelerators. After acceleration a "beam transport line" (also called "beamline" and "magnetic channel") is used to guide the beam of high energy protons (and in general of hadrons) to the Dose Delivery System (DDS) and, finally, the patient.

It is equally known that for proton therapy therapeutic beams of relatively low current (of the order of one nanoampere) are used, with energies that correspond to ranges in water between 30 mm and 350 mm. The relevant kinetic energies are in the range 60 to 250 MeV.

Many companies offer "turnkey" centers for proton therapy and some others centers for heavier ions. Some of these centers feature many treatment rooms while in others the accelerated beam feeds a single room.

In all instances a beam transport line - made of dipoles (which are beam bending elements), quadrupoles (which are beam focusing elements) and other magnetic or electric elements - guides the beam from the accelerator to a DDS and, through it, to the point in which the patient is located, usually called an "isocenter".

An "active" DDS typically contains at least three components:

i. two Scanning Magnets (SMx and SMy), located typically at least 2 meters upstream of the isocenter, which bend the beam transversally on a treatment area of about 300 mm × 300 mm;

ii. a set of beam monitors placed about 500 millimetres upstream of the isocenter;

iii. a vacuum chamber that is connected with the vacuum chamber of the beamline, that connects the accelerator to the DDS, and typically terminates with a thin window just before the monitors, so to reduce the beam widening due to multiple scattering.

It is also known that the elements of a beam transport line are either mounted on supports placed on the floor (to form a fixed horizontal or vertical or inclined beam transport line) or on a rigid mechanical structure that rotates around the patient. Usually such a rotating beam transport line is called a "gantry".

The DDS is integrated in the last part of the beam transport line. In some embodiments both Scanning Magnets are "downstream" of the last magnet of the beam transport line and in others at least one of the two Scanning Magnets is located "upstream" of the last element of the beam transport line.

A Complex comprising an accelerator, a beam transport line and a DDS can be called a "proton-beam emitting device" and more generally a "hadron-beam emitting device".

At any time the currents and the voltages applied to the magnetic and electric elements of such a particle emitting device are defined by the Control System that, during a therapy session, receives signals from sensors distributed on all the hardware elements and sends signals to the corresponding actuators to realize, step-by-step, the irradiation plan defined by the Treatment Planning System (TPS).

It is known that at the isocenter a radiation therapy beam is characterized mainly by the point of entry and the direction in which it enters the patient body, the average kinetic energy E of the particles beam, the energy spread ΔE (usually defined by the Full Width at Half Maximum of the energy distribution), the current I (typically averaged on times of less than 0.1 ns), which is a function of time, and the two transverse Full Width at Half Maximum FWHMx and FWHMy of the beam in the x-y plane, which is perpendicular to the beam direction.

Multiple coulomb scattering in the materials traversed by the particles, before arriving at the isocenter, and in the thicknesses of the monitors placed upstream of the patient, contribute to the values FWHMx and FWHMy measured "in air and at the isocenter". These effects must be minimized.

By tuning the currents in the elements of the beam transport line the Control System of the ion-beam emitting device adjusts the transverse widths in a typical range that goes from 3 mm to 20 mm.

An almost mono-energetic beam of hadrons deposits the maximum of the dose in the well-known "Bragg peak", which is located at a depth R in the target. In water targets and soft tissues targets, the range R (measured in mm) for protons can be approximately computed as a function of the energy E (measured in MeV) using the equation:

$$R = 0.0215 \, E^{1.77} \qquad\qquad (eq.1)$$

A similar formula applies to larger charge and mass particles.

Geometrically, during a therapeutic treatment, a pencil beam of energy E is characterized by a "beam spot" caused by the superposition of the Bragg peaks of the beam particles, in which the largest dose density is deposited. Such beam spot is centered at a depth R and has a "longitudinal" Full Width at Half Maximum (FWHM) Wd and two "transverse" Full Widths at Half Maximum Wx and Wy.

In the case of protons, the "longitudinal" Full Width at Half Maximum of the peak Wd (in mm) can be approximately written as

$$Wd = 12 \, mm \, (E/Emax) \qquad\qquad (\text{with } Emax = 230 \, MeV).$$

It is well known that Multiple Coulomb Scattering of the ions in the target widens the dimensions of the pencil beam so that the actual transverse widths Wx and Wy are obtained by combining quadratically FWHMx and FWHMy with the width FWHM(MS) due to multiple scattering inside the patient body.

During a treatment the beam spot, that actively treats the target, is moved longitudinally in the patient body, by varying the beam energy E, and transversally on the treatment area, by acting on the currents Ix and Iy powering the Scanning Magnets SMx and SMy.

The TPS determines the optimal position of the "planned spots" in the patient body and, for each of them, the number of particles of energy E that have to be sent in that direction to obtain the desired dose distribution in the target while sparing the organs at risk (OARs).

The TPS typically utilizes as input the electron densities of the tissues traversed by the particles, because the energy lost per unit length by a charged particle is proportional to the local electron density. In practice often the greyscale CT values, or Hounsfield numbers of Computer Tomography (CT) images (i.e. the amount of local greyness), determines the local electron densities, which are used to correct the ranges of the particles penetrating the patient body along well-defined paths. As an output, for every incoming direction the TPS typically builds a "distorted" water target, immersed in water, which is such that the beam energy E needed to "visit" any particular point in the target with the beam spot, i.e. a planned spot, is obtained by the TPS from a set of measured ranges of particles in water.

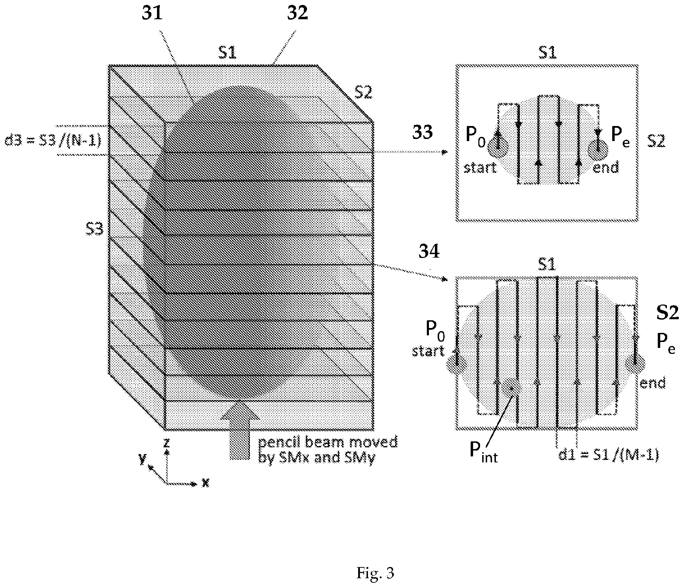

In a typical "Spot Scanning" technique, described for example in E. Pedroni et al, The 200 MeV proton therapy project at the Paul Scherrer Institute: conceptual design and practical realization, Medical Physics, 22(1), (1995) 37, and shown in Figure 3, a water target 31 is a squeezed ellipsoid immersed in water.

The target 31 is inscribed in a parallelepiped 32 - having sides S1, S2 and S3 - and the planned spots are contained in N Equal Energy Layers (EELs) that are perpendicular to the z-direction. In the figure the layers are separated by a distance d3 =S3/(N-1), but in practice the planes are not always equidistant and not all of them are equally visited by the beam spot because the particles that visit distal planned spots indirectly irradiate the traversed tissues thus reducing the dose to be deposited by direct visits.

During a therapeutic treatment the beam spot is moved, by powering the Scanning Magnets with the current Ix and Iy, to a starting position Po(xo, yo) determined by the TPS. As shown in box 33 of figure 3, based on the TPS, the Control System acts on the components of the beam transport line and controls the beam spot in the x,y plane delivering the dose till the prescribed dose value has been reached. Once dose delivery has been terminated, the beam-spot is moved to the next transverse position, e.g. the end position Pe(xe, $y_e$) in the box 33 of figure 3.

Usually, after a layer is scanned with particles of energy E1, the energy is either decreased or increased to a value E2, which may be different from E1 by a fractionally small amount, and a contiguous EEL is scanned transversally in the x and y directions. This is shown in box 34 of figure 3, where delivery of a predetermined dose is foreseen in three beam-spot: a starting spot $P_0(x_0, yo)$, an intermediate spot $P_{int}(x_{int}, yint)$ and an end spot $P_e(x_e, y_e)$. The beam spot is moved from the start spot to the intermediate one by moving through transversal steps d1= S1/(M-1), with M being a positive integer bigger than 1.

In another less used procedure, after the visit to a planned spot is completed, i.e. the planned dose is delivered, the energy is changed and another planned spot is visited that has the same transverse coordinates x and y but a different depth coordinate. This "longitudinal" Spot Scanning procedure may be used, for example, when the time needed to change energy is short with respect to the time necessary to scan an EEL.

It is known that to obtain a uniform dose distribution it is advantageous if the distance, in the transverse plane, between the centres of the planned spots is equal to about 75% of the transverse Full Width at Half Maximum. For protons at a water-depth R = 200 mm the Multiple Scattering width is FWHM(MS) = 10 mm that, combined quadratically with the widths in air at the isocenter (for instance, FWHMx = FWHMy = 7 mm), gives beam widths in the patient body Wx = Wy = 12 mm, which imply a transverse distances between contiguous planned spots equal to $0.75 \times 12 = 9.2$ mm.

A technique used to improve the uniformity of the target dose distribution is "rescanning", also called "repainting", described for example in Bernatowicz et al., "Comparative study of layered and volumetric rescanning for different scanning speeds of proton beam in liver patients", Phys. Med. Biol., 58 (2013) 7905-7920), allows for, in successive rescannings, the correction of statistical and systematic errors in which the system could have occurred. In this paper it has been demonstrated that excellent results are obtained if the number of rescannings is not smaller than 5.

In the therapy with protons and other ions, a problem that is felt is the correct irradiation of organs that move, in particular for the respiration cycle but also for other causes. From this point of view the treatment of cardiac arrhythmias is particularly difficult, as described, for instance, in H. I. Lehmann, D. L. Packer et al, "Feasibility Study on Cardiac Arrhythmia Ablation Using High-Energy Heavy Ion Beam", Scientific Reports, Vol. 6, 2016, Article number: 38895.

A solution is the irradiation of moving targets with the "gating" technique in which the beam current is switched on only when the point in which the dose has to be deposited is - usually in the end-exhale phase - along the path of the pencil beam. This procedure has many inconveniences, in particular because of a need for longer irradiation times.

An alternative, more effective, solution is tumour tracking. As is known, in this procedure a monitoring device determines the in-beam position and shape of the target at any instant during the irradiation and the Control System of the ion-beam emitting device adjusts in real time the coordinates of the centre of the beam spot to target always the same planned spot cancelling the effects of the movements.

The follow up of the target movements may be performed in the transverse plane by keeping the energy E of the beam fixed and using SMx and SMy and a "2-dimensional feedback" system based on the measurements done by the position-measuring devices. Longitudinal follow-up is more difficult because the energy has to be rapidly adjusted, for instance when treating a lung tumour and a rib enters in the trajectory of the hadrons pencil beam due to respiration movements.

The time needed to vary the beam energy changes with the type of accelerator.

Cyclotrons and synchrocyclotrons typically accelerate 10-20 nanosecond particles "pulses" separated by 100-200 ns during which the current is zero. The energy of the beam is typically always the same and, in an Energy Selection System (ESS), movable absorbers reduce the energy of the beam to the value needed to reach the depth where, according to the TPS, the Bragg peak has to be placed. The minimum times for moving the absorbers, and thus change energy, are of the order of 50-100 milliseconds.

Synchrotrons accelerate particles in cycles: the time to reach the needed energy E is typically one second and the beam is extracted, during the "flat-top" of the bending magnetic field versus time, directly at the needed energy. In this mode, beam pulses last from a second to many seconds. However, the National Institute for Radiological Sciences (NIRS - Japan) has pioneered the extraction of beams of different energies within the same machine cycle, by allowing the remaining beam after the first extraction to be directly brought to the next desired energy (Y. Iwata et al., "Multiple-energy operation with extended flattops at HIMAC", Nucl. Instr. Meth. A 624 (2010) 33). The beam extraction is obtained with transverse RF-knockout (as described, for instance, by T. Furukawa et al., "Global spill control in RF- knockout slow extraction", Nucl. Instr. Meth. A 522 (2004) 196) and the switch time between Equal Energy Layers is reduced to about 100 milliseconds.

For synchrotrons another possibility is disclosed by G. Kraft el al. in US 2013/0092839 "Particle-beam generating device": movable absorbers are used to change the energy in steps. The time needed is again of the order of about 100 milliseconds.

Proton and other light ions linacs produce pulses that are around 2-5 microseconds long and are separated by a thousand times longer time interval during which there is no beam, since the typical repetition rates are around 200-400 Hz. The special feature of linacs (described in U. Amaldi, S. Braccini, P. Puggioni in "High Frequency Linacs for Hadron Therapy", Rev. Acc. Sci. Tech., Vol.2, 2009, 111-131) is that by acting on the input power and phase of the klystrons powering the separate, successive accelerating modules, the energy can be varied in about 2 milliseconds. This method, being electronically controlled, is a far more reliable method of controlling beam energy than the methods that use mechanically movable energy absorbers. Moreover, according to the repetition rate (200-400 Hz) the time needed to change the energy and the number of particles in the pulse is in the range 2.5-5 milliseconds, more than 10 times shorter than what is needed when the protons, and other nuclear particles, are accelerated by (synchro)cyclotrons or synchrotrons.

Typically, a beam transport line is used to guide particles from the accelerator section to the DDS, or other output section of the radiotherapy device. US8173981B2 describes a Fixed Field Alternating Gradient (FFAG) structure using permanent magnets and which can be used as a beam transport line or a gantry with a large energy acceptance. This structure can efficiently transmit beams having energies that differ by 10% and more. W. Wan et al, "Alternating-gradient canted cosine theta superconducting magnets for future proton gantries", Phys. Rev. ST Accel. Beams, Vol 18/10 (2015) 103501, describes a solution comprising superconducting magnets. This latter solution has been improved by L. Brower et al, "Design of an achromatic superconducting magnet for a proton therapy gantry", IEEE Trans. Appl. Superconductivity, Vol 27/4, 2016, 4400106, which also provides a large energy acceptance. US15542383 discloses the design of relevant superconducting magnets. However, both solutions are difficult to build and are costly.

EP3178522 discloses a particle therapy system for irradiating a target with a scanning beam technique. The system comprises an irradiation planning device with a planning algorithm configured to associate a particle beam energy E(i) to each spot of the irradiation plan and organize the spots in a sequence of spots according to energy. The system comprises a control system configured for controlling in parallel, from spot to spot, a variation of an output energy of a beam generator, a variation of a magnetic field of one or more electromagnets of a beam transport system and a variation of a magnetic field of the scanning magnet.

US2010012859A1 discloses a method for treating or irradiating a target volume with a charged particle beam, to the target volume being associated three coordinates according to a X, Y and Z direction, the Z coordinate corresponding to the beam direction while the X and Y coordinates correspond to directions perpendicular to the Z direction. The charged particle beam produces in the target volume an irradiation spot. The method comprises the following steps: continuously scanning the spot in the X and Y directions and applying a continuous movement to said spot in the Z direction by seamlessly modifying the energy of said beam, the step of applying a continuous movement in Z direction and the step of continuous scanning in X and Y direction being performed simultaneously, thereby performing a continuous 3D scanning of the target volume.

## OBJECTS AND SUMMARY OF INVENTION

It is therefore an object of the present invention to solve the problems of the prior art devices for emitting particle beams like protons and other ions beams.

In particular, it is an object of the present invention to allow a rapid change of beam energy, so as to reduce treatment times and improve irradiation efficiency in moving targets.

These and other object of the present invention will be made clearer by the following description and the attached claims, which form an integral part of this description.

According to a first aspect, the invention relates to a beam transport line for a particle (in particular protons) accelerator, adapted to guide the proton beam from the accelerator to a dose delivery system, and comprising a plurality of electromagnetic elements. At least one of these electromagnetic elements is connected to either a 2-quadrant or a 4-quadrant power supply unit arranged to produce a current that can be varied by the Control System in less than 50 milliseconds so that, when the beam energy is varied to move the beam spot along the beam direction, the beam is kept focused on a target with losses along the beam transport line and the dose delivery system not larger than 10% and variations of the transverse Full Widths at Half Maximum of the beam spot not larger than 10%.

A beam transport line with these features allows to move the beam-spot very quickly, and to realize radiation therapy systems in which the energy of the beam is varied very quickly. This allows to reduce treatment times and improve irradiation efficiency in moving targets.

According to a second aspect, the invention is directed to a beam transport line for an accelerator of ions, having electric charge Z between 2 and 10 and mass number A between 4 and 20, arranged to guide the ion beam from the accelerator to a dose delivery system. The beam transport line comprises a plurality of electromagnetic elements, such as quadrupoles or deflecting magnets to guide the ion beam. At least one of these electromagnetic elements is connected to either a 2-quadrant or a 4-quadrant power supply unit arranged to produce a current that can be varied by the Control System in less than 50 milliseconds so that, when the beam energy is varied to move the beam spot along the beam direction, the beam is kept focused on a target with losses along the beam transport line and the dose delivery system not larger than 10% and variations of the transverse Full Widths at Half Maximum of the beam spot not larger than 10%.

The beam transport line with 2-quadrant or 4-quadrant power supply units and the above-mentioned features, therefore, allows to reduce treatment time of the patient both in the case of proton beams and in the case of ion beams.

Advantageously, the control system that controls the currents circulating in the electro-magnetic elements of the beam transport line, also controls the accelerator that generates the particle beam and in particular controls the energy of the particle beam emitted by the accelerator. The control system is then configured so as to control the currents of the power supply units as a function of the variation of energy of the accelerator, so as to allow tracking of moving targets, such as a beating heart.

Furthermore, according to a further aspect, the invention is directed to a radiotherapy system including a particle accelerator, in particular protons or ions with an atomic number between 4 and 20 and an electric charge between 2 and 10, and a beam transport line - having the features listed above and better detailed below - adapted to guide the beam of particles coming out of the accelerator.

In one embodiment, the radiotherapy system further comprises a dose distribution system, and the beam transport line is adapted to guide the particles (protons or ions) beam out of the accelerator to the dose distribution system.

In particular, in one embodiment the radiation therapy system comprises a particle (in particular protons, but more generally hadrons) accelerator adapted to emit a particles beam, and a beam transport line comprising a plurality of electromagnetic elements to guide the particles beam from the accelerator to a dose distribution system. A control system is operatively connected to the accelerator to vary the energy of the particle beam and to control the beam transport line. At least one of the electromagnetic elements is connected to either a 2-quadrant or a 4-quadrant power supply unit arranged to produce a current that can be varied by the Control System in less than 50 milliseconds so that, when the beam energy is varied to move the beam spot along the beam direction, the beam is kept focused on a target with losses along the beam transport line and the dose delivery system not larger than 10% and variations of the transverse

Full Widths at Half Maximum of the beam spot not larger than 10%.

## BRIEF DESCRIPTION OF DRAWINGS

Further features and advantages of the present invention will be clearer from the following detailed description of some preferred embodiments, which is made with reference to the annexed drawings.

The different characteristics described with reference to the individual embodiments can be combined as desired, if one would like to make use of advantages resulting specifically from a particular combination.

In these drawings,

- Figure 1 illustrates a beam transport line according to an embodiment of the present invention;

- Figure 2 illustrates a variant of the beam transport line of Figure 1;

- Figure 3 schematically illustrates a target in water and a known method of controlling a beam of protons and other light ions for depositing a dose of protons on the target.

## DETAILED DESCRIPTION OF THE INVENTION

In the following description, identical reference numbers or symbols are used to illustrate the figures to indicate components with the same function. In addition, for clarity of illustration, some references may not be repeated in all figures. While the invention is susceptible of various modifications and alternative constructions, some preferred embodiments are shown in the drawings and will be described in details herein below. It should be understood, however, that there is no intention to limit the invention to the specific disclosed embodiment but, on the contrary, the invention intends to cover all the modifications and alternative constructions that fall within the scope of the invention as defined in the claims.

The use of "for example", "etc.", "or" denotes non-exclusive alternatives without limitation, unless otherwise noted. The use of "includes" means "includes, but not limited to", unless otherwise noted.

In the following beam transport lines (also called "beamlines" and "magnetic channels") are disclosed which comprise electric and magnetic elements as bending magnets and quadrupoles, which connect an accelerator of protons and other ions to a Dose Delivery System (DDS) for therapeutic and palliative purposes for treating tumours, and other pathologies which benefit from radiotherapy, for example arteriovenous and other localized arterial malformations, cardiac arrhythmias, renal denervation etc. The magnetic elements are typically electromagnets.

The beam transport line may be part of a gantry or may be a fixed beam transport line.

Some of the beam transport line magnetic elements, for example dipoles or quadrupoles, are connected to rapidly varying power supplies, in particular rapidly varying 2-quadrant and/or 4-quadrant power supplies, so that the proton beam is transported and kept focused when, in less than 50 ms, in particular in the range between 0.1 ms and 50 ms, the energy of the beam is decreased or increased. The current change may be within a fractional interval that goes from about $\pm 1\%$ to about $\pm 10\%$ of their values, but this interval may also increase. The rapid variation of the energy implies that the beam spot can be moved, in less than 50 milliseconds, back and forth by many millimetres, and even centimetres.

This feature is particularly important when a monitoring device detects in real time the 3-dimensional movements of the target in the patient body (due to respiration, heart beating and other body movements) and the Control System of the accelerator-beamline-DDS Complex applies a 3-dimensional feedback system to follow the target, without interrupting the irradiation, thus performing what can be called a "Fast Adaptive Spot Scanning Therapy" - FASST. The invention here disclosed is relevant for the more advanced technique of irradiating moving targets called "tumour tracking". It can also be used to correct the beam spot position in case of a sudden movement of the patient.

In the following, therefore, will be described beamlines - which guide the particles from a proton-beam or other ions-beam therapy accelerator to a DDS - in which at least one magnetic element is connected to either a 2-quadrant or a 4-quadrant power supply producing in the element a current, i.e. a magnetic field, that can be varied by the control system in less than about 50 milliseconds. For achieving such short times, the inductances of the powered coils are matched to the needs.

These beam transport lines have preferred application to accelerators that can rapidly vary the energy of the beam spot - i.e. rapidly vary the position of the Bragg peak inside the target - and is of particular advantage when a target position-measuring device detects, during an irradiation, the necessity of a longitudinal adjustment of the range (i.e. of the energy) of the beam. A longitudinal feedback system, known per se and therefore not described, is preferably combined with a transverse 2-dimensional feedback system and transforms it into a fully "3-dimensional feedback system".

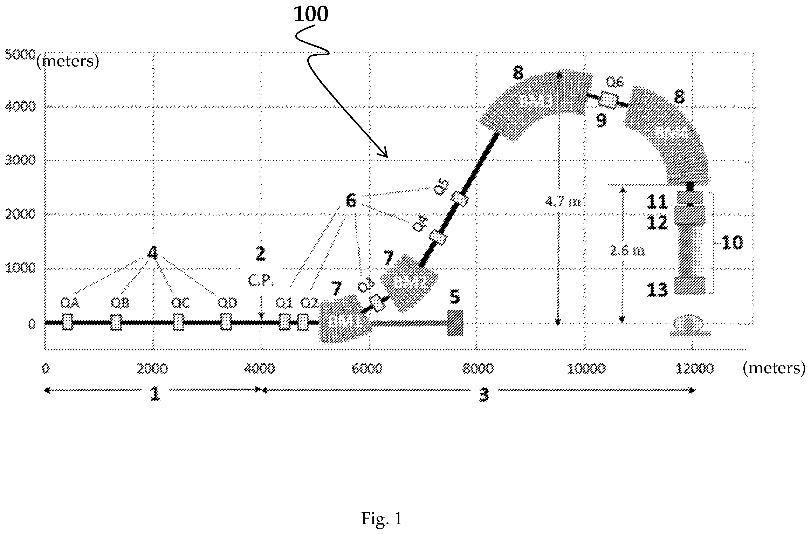

With reference to Figure 1, a beam transport line 100 is illustrated that can connect a proton accelerator, in particular a linac (not shown in the figure) capable of emitting a beam with energy up to 230 MeV, to a dose distribution system 10.

The beam transport line 100 comprises a horizontal beam transport line 1 and a rotating beam transport line 3. The horizontal beam transport line 1 and the rotating beam transport line 3 are connected at a "coupling point" C.P., indicated by reference number 2.

At the coupling point, a fixed vacuum chamber of the horizontal beam transport line 1 is connected, by means of a gas-tight connection, to a rotating vacuum chamber of the rotating beam transport line 3.

In the example in Figure 1, the horizontal beam transport line 1 comprises four quadrupoles QA, QB, QC and QD, designated by reference number 4, while the rotating beam transport line 3 comprises six quadrupoles Q1-Q6, designated by reference number 6, and four bending magnets BM1-BM4, designated by reference number 7.

In this example, quadrupoles 4 and 6 and bending magnets 7 of the beam transport line 100 are connected to 2-quadrant switch-mode power supply units (not shown) that can vary their currents by about $\pm$ 10% in short times, i.e. about 2 milliseconds.

In other embodiments and for some magnets (in particular the quadrupoles) it may be convenient to use 4-quadrant switch-mode power supply units. The reason is that the magnetic fields of the bending magnets never reverse sign so that for them 2-quadrant switch mode power supply units are sufficient. Instead it could happen that, when varying the beam energy, the fields' direction in some quadrupoles have to be inverted; in such instances 4-quadrant switch mode power supply units are needed. This is not the case for the quadrupoles of the embodiment of Figure 1.

According to a preferred embodiment, therefore, the main components of the system of figure 1, which transports protons with E(max) = 230 MeV, are:

1. an "horizontal beam transport line", which is preferably, but not necessarily, 4 m long and guides the beam from the end of the proton linac to the coupling point C.P.;

2. the "coupling point" C.P. between the fixed vacuum chamber and the rotating vacuum chamber;

3. the "rotating beam transport line" (usually called "gantry") which is about 8 m long, and its magnetic elements;

4. the "standard quadrupoles" QA, QB, QC and QD forming the last part of the beamline;

5. the "beam dump";

6. the "standard quadrupoles" Q1, Q2....Q5 mounted on the rotating gantry;

7. the "30-degree bending magnets" BM1 and BM2 with a maximum magnetic field B(max) = 1.6 tesla;

8. the "75-degree bending magnets" BM3 and BM4 with a maximum magnetic field B(max) = 1.6 tesla;

9. the "large-aperture quadrupole" Q6;

10. the "Dose Distribution System" DDS;

11. the "scanning magnet SMx" that bends the beam in the x-direction;

12. the "scanning magnet SMy" that bends the beam in the y-direction;

13. the "beam monitors".

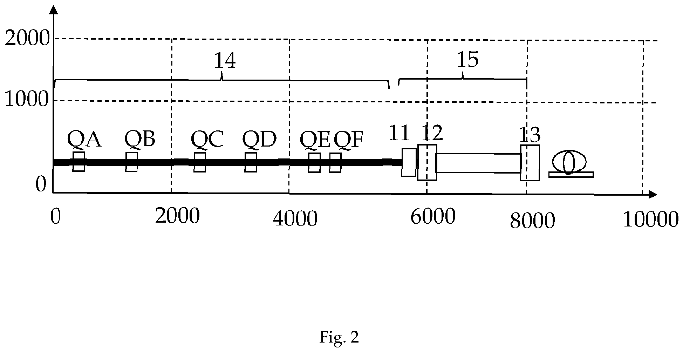

A simpler embodiment is shown in Figure 2, where reference number 14 indicates an horizontal beam transport line and reference number 15 indicates an horizontal DDS. In the example of Figure 2 the horizontal beam transport line comprises six quadrupoles QA, QB, QC, QD, QE and QF, while the DDS 15 comprises the two scanning magnets SMx and SMy, indicated with the references 11 and 12, and the beam monitors 13.

The embodiment of Figure 1 provides small emittances of the proton beam emitted and therefore the magnets have small iron-to-iron gaps: 40 mm for the standard quadrupoles and 30 mm for the bending magnets. The magnets are much lighter than in other existing gantries. Moreover, they form an integral part of the supporting structure such that the overall weight of the gantry (with all its elements) is only about 25 tons. The whole system can be attached to the wall of the shielded treatment room.

The accelerator-beam transport line-DDS of Figure 1 runs at a frequency comprised between 100 Hz and 500 Hz, preferably at 200 Hz, i.e. its energy can be varied every 2-10 milliseconds, preferably every 5 milliseconds. The control of variation of the beam energy is implemented by the control system (not shown in figure 1) which acts on the accelerator to vary the beam energy and thus adjust the longitudinal position of the beam-spot.

The maximum percentage variations of the magnetic fields in the bending magnets BM1, BM2, BM3, and BM4 have been computed from the above equation (eq. 1) by requiring that, at each energy, the depth of the Bragg peak can be moved back and forward by twice its width Wd. With the above approximate equation this means a maximum range variation

$$\Delta R(max) = \pm 24 \text{ mm } (E/230).$$

This is achieved in a few milliseconds by varying the currents circulating in the coils of the deflecting magnets thanks to the 2-quadrant or 4-quadrant switched mode power supply units.

Table 1 below shows some simulations for the system of figure 1. In column C2 of Table 1 the energy corresponding to the ranges of column C1 are listed, according to the first equation quoted above. The needed maximum range variations $\Delta R(max)$ are listed in Column 4 of Table 1. The last column gives the maximum variations of the fields in the bending magnets that give the range adjustments $\Delta R(max)$ of Column 4.

Table 1

| C1 Range R [mm] | C2 Energy E [MeV] | C3 Max range step $\Delta R$ (max) [mm] | C4 Max energy step $\Delta E$ (max) [MeV] | C5 Maximum field variation ($\Delta B/B$) |
|---|---|---|---|---|
| 40 | 69 | $\pm$ 7.20 | $\pm$ 6.72 | $\pm$ 5.11% |
| 70 | 95 | $\pm$ 9.91 | $\pm$ 7.32 | $\pm$ 4.20% |
| 100 | 116 | $\pm$ 12,1 | $\pm$ 7.63 | $\pm$ 3.62% |
| 150 | 147 | $\pm$ 15.3 | $\pm$ 8.16 | $\pm$ 3.12% |
| 200 | 172 | $\pm$ 18.0 | $\pm$ 8.38 | $\pm$ 2.76% |
| 250 | 196 | $\pm$ 20.4 | $\pm$ 8.70 | $\pm$ 2.54 % |
| 300 | 217 | $\pm$ 22.6 | $\pm$ 8.88 | $\pm$ 2.40% |
| 320 | 225 | $\pm$ 23.5 | $\pm$ 8.93 | $\pm$ 2.32 % |

When the proton energy increases from 70 MeV to 230 MeV the percentage variations of the fields in the bending magnets decrease from about $\pm$ 5% to about $\pm$ 2% without ever changing direction.

Beam dynamics calculations show that also in the quadrupoles the directions of the magnetic fields do not change sign, so that for the embodiment of Fig. 1 2-quadrant power supplies are sufficient. The same statement is valid for the embodiment of Fig. 2. However, in other embodiments the dynamics of the beams are different and it may be necessary to use 4 quadrant switch mode power supplies.

From the above description it can be seen that the teaching of this invention efficiently solves the proposed objects and obtains the advantages indicated.

People skilled in the art can introduce modifications or variations without leaving the scope of protection of the present invention as described and claimed.

As an example, the dimensions of the beamlines described above with reference to Figures 1 and 2 are preferred but not mandatory.

In a particularly advantageous embodiment, the rapid longitudinal tracking of the tumour according to the invention can be combined with rescanning or repainting, for example with 5 times repeated scans. This combination offers the best method for accurately irradiating a moving target.

It is also clear that the beam transport lines, as described and claimed, can also be used whenever the beam energy is changed rapidly and can result in shorter treatment times. This is because by using fast 2- and/or 4-quadrant switch mode power supply units, the magnetic elements in the beam transport line are able to adapt more quickly to energy changes in the beam.

In order to follow the energy of a proton therapy beam the present invention modifies the focusing and bending properties of a beamline by using rapidly varying 2-quadrant and 4-quadrant power supplies, possibly working in the well-known switch-mode. At present the fractional current variations of such rapid power supplies have soft limits of about ± 10% and this determines the limit quoted above. However, this band of variation, which may increase, is more than sufficient for the 3-dimensional tracking of a target.

The rapid changes of the currents powering the interested magnetic elements are preferably synchronous and cause small proton losses along the beamline, preferably losses less than 10%. Moreover, the transverse FWHMs of the beam spot in the target have to vary by less than ± 10%.

In conclusion, a beam transport line with a large energy acceptance is not required, because when the beamline electromagnets are driven by either a rapid 2-quadrant or a rapid 4-quadrant power supply the beam transport line can efficiently transmit beams of varying energy.

**Claims**

1. A beam transport line (100, 14) for a proton accelerator, arranged to guide the proton beam from the accelerator to a dose delivery system (10, 15), wherein the accelerator, the proton beam transport line (100, 14) and the dose delivery system are arranged to be controllable by a control system, and wherein the beam transport line (100, 14) comprises a plurality of electromagnetic elements (4,6,9), **characterized in that** at least one of these electromagnetic elements (4,6,9) is connected to either a 2-quadrant or a 4-quadrant power supply unit arranged to produce a current that can be varied by the control system in less than 50 milliseconds so that, when the beam energy is varied to move the beam spot along the beam direction, the beam is kept focused on a target with losses along the beam transport line (100, 14) and the dose delivery system (10, 15) not larger than 10% and variations of the transverse Full Widths at Half Maximum of the beam spot not larger than 10%.

2. A beam transport line (100, 14) for an accelerator of ions, having electric charge Z between 2 and 10 and mass number A between 4 and 20, arranged to guide the ion beam from the accelerator to a dose delivery system (10, 15), wherein the accelerator, the beam transport line (100, 14) and the dose delivery system (10, 15) are arranged to be controllable by a control system, and wherein the beam transport line (100, 14) comprises a plurality of electromagnetic elements (4,6,9), **characterized in that** at least one of these electromagnetic elements (4,6,9) is connected to either a 2-quadrant or a 4-quadrant power supply unit arranged to produce a current that can be varied by the control system in less than 50 milliseconds so that, when the beam energy is varied to move the beam spot along the beam direction, the beam is kept focused on a target with losses along the beam transport line (100, 14) and the dose delivery system (10, 15) not larger than 10% and variations of the transverse Full Widths at Half Maximum of the beam spot not larger than 10%.

3. The beam transport line according to claim 1 or 2, **characterized in that** the accelerator is an ion linac.

4. The beam transport line according to claim 1 or 2, **characterized in that** the accelerator is a ion synchrotron, either superconducting or at room temperature.

5. The beam transport line according to claim 1 or 2, **characterized in that** the accelerator is either a cyclotron or a synchrocyclotron.

6. A radiotherapy system comprising the beam transport line of either claim 1 or claim 2.

7. A radiation therapy system, comprising

a particle accelerator adapted to emit a particle beam, said particle beam being a beam of protons or a beam of ions with an electric charge between 2 and 10 and a mass number between 4 and 20,
a beam transport line (100, 14) comprising a plurality of electromagnetic elements (4,6,9) to guide the particles beam from the accelerator,
a control system operatively connected to the accelerator to vary the energy of the particle beam,
**characterized by** the fact that
at least one of the electromagnetic elements (4,6,9) is connected to either a 2-quadrant or a 4-quadrant power supply unit arranged to produce a current that can be varied by the control system in less than 50 milliseconds so that, when the beam energy is varied to move the beam spot along the beam direction, the beam is kept focused on a target with losses along the beam transport line (100, 14) and the dose delivery system (10, 15) not larger than 10% and variations of the transverse Full Widths at Half Maximum of the beam spot not larger than 10%.

8. The system according to claim 7, further comprising a dose distribution system (10,15), wherein the beam transport line is adapted to guide the particles beam from the accelerator to the dose distribution system.

9. The radiotherapy system according to claim 7 or 8, wherein the control system is adapted to vary the accelerator energy every 2 - 10 milliseconds and to control the beam transport line (100, 14) and the dose distribution system (10,15) with a frequency of 100 - 500 Hz.

10. The radiotherapy system according to any of claims from 7 to 9, wherein the accelerator is a linac for ions.

## Patentansprüche

1. Strahltransportlinie (100, 14) für einen Protonenbeschleuniger, die derart eingerichtet ist, dass sie den Protonenstrahl von dem Beschleuniger zu einem Dosisabgabesystem (10, 15) führt, wobei der Beschleuniger, die Protonenstrahl-Transportlinie (100, 14) und das Dosisabgabesystem derart angeordnet sind, dass sie durch ein Steuersystem steuerbar sind, und wobei die Strahltransportlinie (100, 14) eine Vielzahl von elektromagnetischen Elementen (4, 6, 9) umfasst, **dadurch gekennzeichnet, dass** mindestens eines dieser elektromagnetischen Elemente (4, 6, 9) entweder mit einer 2-Quadranten- oder einer 4-Quadranten-Stromversorgungseinheit verbunden ist, die derart eingerichtet ist, dass sie einen Strom erzeugt, der durch das Steuersystem in weniger als 50 Millisekunden variiert werden kann, so dass der Strahl, wenn die Strahlenergie variiert wird, um den Strahlfleck entlang der Strahlrichtung zu bewegen, auf einem Target fokussiert gehalten wird, wobei die Verluste entlang der Strahltransportlinie (100, 14) und des Dosisabgabesystems (10, 15) nicht größer als 10% und die Schwankungen der transversalen Halbwertsbreiten des Strahlflecks nicht größer als 10% sind.

2. Strahltransportlinie (100, 14) für einen Ionenbeschleuniger mit einer elektrischen Ladung Z zwischen 2 und 10 und einer Massenzahl A zwischen 4 und 20, die derart eingerichtet ist, dass sie den Ionenstrahl von dem Beschleuniger zu einem Dosisabgabesystem (10, 15) führt, wobei der Beschleuniger, die Strahltransportlinie (100, 14) und das Dosisabgabesystem (10, 15) derart eingerichtet sind, dass sie durch ein Steuersystem steuerbar sind, und wobei die Strahltransportlinie (100, 14) eine Vielzahl von elektromagnetischen Elementen (4, 6, 9) umfasst, **dadurch gekennzeichnet, dass** mindestens eines dieser elektromagnetischen Elemente (4,6, 9) entweder mit einer 2-Quadranten- oder einer 4-Quadranten-Stromversorgungseinheit verbunden ist, die derart eingerichtet ist, dass sie einen Strom erzeugt, der durch das Steuersystem in weniger als 50 Millisekunden variiert werden kann, so dass, wenn die Strahlenergie variiert wird, um den Strahlfleck entlang der Strahlrichtung zu bewegen, der Strahl auf einem Target fokussiert gehalten wird, wobei die Verluste entlang der Strahltransportlinie (100, 14) und dem Dosisabgabesystem (10, 15) nicht größer als 10% sind und die Schwankungen der transversalen Halbwertsbreiten des Strahlflecks nicht größer als 10% sind.

3. Strahltransportlinie nach Anspruch 1 oder 2, **dadurch gekennzeichnet, dass** der Beschleuniger ein Ionen-Linac ist.

4. Strahltransportlinie nach Anspruch 1 oder 2, **dadurch gekennzeichnet, dass** der Beschleuniger ein Ionensynchrotron ist, der entweder supraleitend oder bei Raumtemperatur betrieben wird.

5. Strahltransportlinie nach Anspruch 1 oder 2, **dadurch gekennzeichnet, dass** der Beschleuniger entweder ein Zyklotron oder ein Synchrozyklotron ist.

**6.** Strahlentherapiesystem, welches die Strahltransportlinie entweder nach Anspruch 1 oder Anspruch 2 umfasst.

**7.** Strahlentherapiesystem umfassend

einen Teilchenbeschleuniger, der zur Emission eines Teilchenstrahls eingerichtet ist, wobei der Teilchenstrahl ein Protonenstrahl oder ein Ionenstrahl mit einer elektrischen Ladung zwischen 2 und 10 und einer Massenzahl zwischen 4 und 20 ist,

eine Strahltransportlinie (100, 14) mit einer Vielzahl von elektromagnetischen Elementen (4, 6, 9) zum Leiten des Teilchenstrahls aus dem Beschleuniger,

ein Steuersystem, das mit dem Beschleuniger wirkverbunden ist, um die Energie des Teilchenstrahls zu variierten,

**dadurch gekennzeichnet, dass**

mindestens eines der elektromagnetischen Elemente (4,6, 9) entweder mit einer 2-Quadranten- oder einer 4-Quadranten-Stromversorgungseinheit verbunden ist, die derart eingerichtet ist, dass sie einen Strom erzeugt, der durch das Steuersystem in weniger als 50 Millisekunden variiert werden kann, so dass, wenn die Strahlenergie variiert wird, um den Strahlfleck entlang der Strahlrichtung zu bewegen, der Strahl auf einem Target fokussiert gehalten wird, wobei Verluste entlang der Strahltransportlinie (100, 14) und dem Dosisabgabesystem (10, 15) nicht größer als 10% und die Schwankungen der transversalen Halbwertsbreiten des Strahlflecks nicht größer als 10% sind.

**8.** System nach Anspruch 7, das ferner ein Dosisverteilungssystem (10, 15) umfasst, wobei die Strahltransportlinie derart eingerichtet ist, dass sie den Teilchenstrahl vom Beschleuniger zum Dosisverteilungssystem leitet.

**9.** Strahlentherapiesystem nach Anspruch 7 oder 8, wobei das Steuersystem derart eingerichtet ist, dass es die Beschleunigerenergie alle 2 - 10 Millisekunden variiert sowie die Strahlentransportleitung (100, 14) und das Dosisverteilungssystem (10, 15) mit einer Frequenz von 100 - 500 Hz steuert.

**10.** Strahlentherapiesystem nach einem der Ansprüche 7 bis 9, wobei der Beschleuniger ein Linac für Ionen ist.

**Revendications**

**1.** - Ligne de transport de faisceau (100, 14) pour un accélérateur de protons, agencée pour guider le faisceau de protons de l'accélérateur à un système de distribution de dose (10, 15), dans laquelle l'accélérateur, la ligne de transport de faisceau de protons (100, 14) et le système de distribution de dose sont agencés pour être commandables par un système de commande, et dans laquelle la ligne de transport de faisceau (100, 14) comprend une pluralité d'éléments électromagnétiques (4, 6, 9), **caractérisée par le fait qu'**au moins l'un de ces éléments électromagnétiques (4, 6, 9) est connecté à une unité d'alimentation électrique soit à 2 quadrants, soit à 4 quadrants agencée pour produire un courant qui peut être modifié par le système de commande en moins de 50 millisecondes de telle sorte que, lorsque l'énergie de faisceau est modifiée pour déplacer le point de faisceau le long de la direction de faisceau, le faisceau est maintenu focalisé sur une cible avec des pertes le long de la ligne de transport de faisceau (100, 14) et du système de distribution de dose (10, 15) qui ne sont pas supérieures à 10 % et des variations des largeurs totales transversales à mi-hauteur du point de faisceau qui ne sont pas supérieures à 10 %.

**2.** - Ligne de transport de faisceau (100, 14) pour un accélérateur d'ions, ayant une charge électrique Z entre 2 et 10 et un nombre de masse A entre 4 et 20, agencée pour guider le faisceau d'ions de l'accélérateur à un système de distribution de dose (10, 15), dans laquelle l'accélérateur, la ligne de transport de faisceau (100, 14) et le système de distribution de dose (10, 15) sont agencés pour être commandables par un système de commande, et dans laquelle la ligne de transport de faisceau (100, 14) comprend une pluralité d'éléments électromagnétiques (4, 6, 9), **caractérisée par le fait qu'**au moins l'un de ces éléments électromagnétiques (4, 6, 9) est connecté à une unité d'alimentation électrique soit à 2 quadrants, soit à 4 quadrants agencée pour produire un courant qui peut être modifié par le système de commande en moins de 50 millisecondes de telle sorte que, lorsque l'énergie de faisceau est modifiée pour déplacer le point de faisceau le long de la direction de faisceau, le faisceau est maintenu focalisé sur une cible avec des pertes le long de la ligne de transport de faisceau (100, 14) et du système de distribution de dose (10, 15) qui ne sont pas supérieures à 10 % et des variations des largeurs totales transversales à mi-hauteur du point de faisceau qui ne sont pas supérieures à 10 %.

**3.** - Ligne de transport de faisceau selon la revendication 1 ou 2, **caractérisée par le fait que** l'accélérateur est un accélérateur linéaire d'ions.

**4.** - Ligne de transport de faisceau selon la revendication 1 ou 2, **caractérisée par le fait que** l'accélérateur est un synchrotron à ions, soit supraconducteur, soit à température ambiante.

**5.** - Ligne de transport de faisceau selon la revendication 1 ou 2, **caractérisée par le fait que** l'accélérateur est soit un cyclotron, soit un synchrocyclotron.

**6.** - Système de radiothérapie comprenant la ligne de transport de faisceau selon la revendication 1 ou la revendication 2.

**7.** - Système de radiothérapie, comprenant :

un accélérateur de particules conçu pour émettre un faisceau de particules, ledit faisceau de particules étant un faisceau de protons ou un faisceau d'ions avec une charge électrique entre 2 et 10 et un nombre de masse entre 4 et 20,
une ligne de transport de faisceau (100, 14) comprenant une pluralité d'éléments électromagnétiques (4, 6, 9) pour guider le faisceau de particules provenant de l'accélérateur,
un système de commande relié de manière fonctionnelle à l'accélérateur pour modifier l'énergie du faisceau de particules,
**caractérisé par le fait que**
au moins l'un des éléments électromagnétiques (4, 6, 9) est connecté à une unité d'alimentation électrique soit à 2 quadrants, soit à 4 quadrants agencée pour produire un courant qui peut être modifié par le système de commande en moins de 50 millisecondes de telle sorte que, lorsque l'énergie de faisceau est modifiée pour déplacer le point de faisceau le long de la direction de faisceau, le faisceau est maintenu focalisé sur une cible avec des pertes le long de la ligne de transport de faisceau (100, 14) et du système de distribution de dose (10, 15) qui ne sont pas supérieures à 10 % et des variations des largeurs totales transversales à mi-hauteur du point de faisceau qui ne sont pas supérieures à 10 %.

**8.** - Système selon la revendication 7, comprenant en outre un système de distribution de dose (10, 15), dans lequel la ligne de transport de faisceau est conçue pour guider le faisceau de particules de l'accélérateur au système de distribution de dose.

**9.** - Système de radiothérapie selon la revendication 7 ou 8, dans lequel le système de commande est conçu pour modifier l'énergie d'accélérateur toutes les 2 à 10 millisecondes et pour commander la ligne de transport de faisceau (100, 14) et le système de distribution de dose (10, 15) avec une fréquence de 100 à 500 Hz.

**10.** - Système de radiothérapie selon l'une quelconque des revendications 7 à 9, dans lequel l'accélérateur est un accélérateur linéaire pour ions.

Fig. 1

Fig. 2

Fig. 3

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 20130092839 A, G. Kraft
- US 8173981 B2
- US 15542383 B
- EP 3178522 A
- US 2010012859 A1

### Non-patent literature cited in the description

- **E. PEDRONI et al.** The 200 MeV proton therapy project at the Paul Scherrer Institute: conceptual design and practical realization. *Medical Physics,* 1995, vol. 22 (1), 37
- **BERNATOWICZ et al.** Comparative study of layered and volumetric rescanning for different scanning speeds of proton beam in liver patients. *Phys. Med. Biol.,* 2013, vol. 58, 7905-7920
- **H. I. LEHMANN ; D. L. PACKER et al.** Feasibility Study on Cardiac Arrhythmia Ablation Using High-Energy Heavy Ion Beam. *Scientific Reports,* 2016, vol. 6
- **Y. IWATA et al.** Multiple-energy operation with extended flattops at HIMAC. *Nucl. Instr. Meth. A,* 2010, vol. 624, 33
- **T. FURUKAWA et al.** Global spill control in RF-knockout slow extraction. *Nucl. Instr. Meth. A,* 2004, vol. 522, 196
- **U. AMALDI ; S. BRACCINI ; P. PUGGIONI.** High Frequency Linacs for Hadron Therapy. *Rev. Acc. Sci. Tech.,* 2009, vol. 2, 111-131
- **W. WAN et al.** Alternating-gradient canted cosine theta superconducting magnets for future proton gantries. *Phys. Rev. ST Accel. Beams,* 2015, vol. 18 (10), 103501
- **L. BROWER et al.** Design of an achromatic superconducting magnet for a proton therapy gantry. *IEEE Trans. Appl. Superconductivity,* 2016, vol. 27 (4), 4400106